# EUROPEAN PATENT SPECIFICATION

(11) **EP 1 666 884 B1**
(45) Date of publication and mention of the grant of the patent: **01.04.2009**
(21) Application number: 04026164.6
(22) Date of filing: 04.11.2004
(51) Int. Cl.: G01N 33/68

(54) **Method for determination of arginine, methylated arginines and derivatives thereof**
Methode zur Bestimmung von Arginin, methylierten Argininen und deren Derivate
Méthode pour la détermination de l'arginine, de l'arginine méthylée et de dérivés

(43) Date of publication of application: 07.06.2006
(73) Proprietor: GermedIQ Forschungs- und Entwicklungsgesellschaft mbH, 22459 Hamburg (DE)
(72) Inventor: Böger, Rainer, 20148 Hamburg (DE); Schwedhelm, Edzard, 20251 Hamburg (DE); Maas, Renke, 22457 Hamburg (DE); Riederer, Ulrich, 21039 Escheburg (DE)
(74) Representative: Kloiber, Thomas

(56) References cited:
- MARTENS-LOBENHOFFER J ET AL: "Simultaneous detection of arginine, asymmetric dimethylarginine, symmetric dimethylarginine and citrulline in human plasma and urine applying liquid chromatography-mass spectrometry with very straightforward sample preparation" JOURNAL OF CHROMATOGRAPHY B: BIOMEDICAL SCIENCES & APPLICATIONS, ELSEVIER SCIENCE PUBLISHERS, NL, vol. 798, no. 2, 25 December 2003 (2003-12-25), pages 231-239, XP004474594 ISSN: 1570-0232
- VISHWANATHAN K ET AL: "Determination of arginine and methylated arginines in human plasma by liquid chromatography-tandem mass spectrometry" JOURNAL OF CHROMATOGRAPHY. BIOMEDICAL APPLICATIONS, ELSEVIER, AMSTERDAM, NL, vol. 748, no. 1, 1 October 2000 (2000-10-01), pages 157-166, XP004224393 ISSN: 0378-4347
- HUANG LAN-FANG ET AL: "Simultaneous determination of L-arginine and its mono- and dimethylated metabolites in human plasma by high-performance liquid chromatography-mass spectrometry." ANALYTICAL AND BIOANALYTICAL CHEMISTRY. OCT 2004, vol. 380, no. 4, October 2004 (2004-10), pages 643-649, XP002320582 ISSN: 1618-2642
- OYA TOMOKO ET AL: "Methylglyoxal modification of protein: Chemical and immunochemical characterization of methylglyoxal-arginine adducts" JOURNAL OF BIOLOGICAL CHEMISTRY, vol. 274, no. 26, 25 June 1999 (1999-06-25), pages 18492-18502, XP002320583 ISSN: 0021-9258

## Description

The present invention is in the field of assay methods, and in particular assay methods for arginine and derivatives thereof.

Arginine (ARG) and methylated derivatives thereof play an important role for the biological available nitric oxide (NO) concentration.

NO is essential in several physiological scenes, i.e. homeostasis of cardiovascular system, host defence, neuronal signalling, cell migration and apoptosis. NO is formed in the endothelium by the constitutive, endothelial isoform of nitric oxide synthase using ARG as substrate. All three isoforms of nitric oxide synthase in humans are inhibited by the endogenous inhibitors monomethyl arginine (MMA) and asymmetric dimethylarginine (ADMA) with ADMA being more important than MMA due to its up to ten times higher concentration in human plasma. ADMA and MMA originate from protein arginine methylation by protein arginine methyltransferases (PRMTs) after protein degradation. In this way, also symmetrical dimethylarginine (SDMA) is produced, which does not exert biological activity, however. Approximately one fifth of the ADMA is renally excreted in man whereas enzymatic degradation by dimethylarginine dimethylaminohydrolase (DDAH) is the major pathway for elimination of ADMA.

A brief scheme is given below:

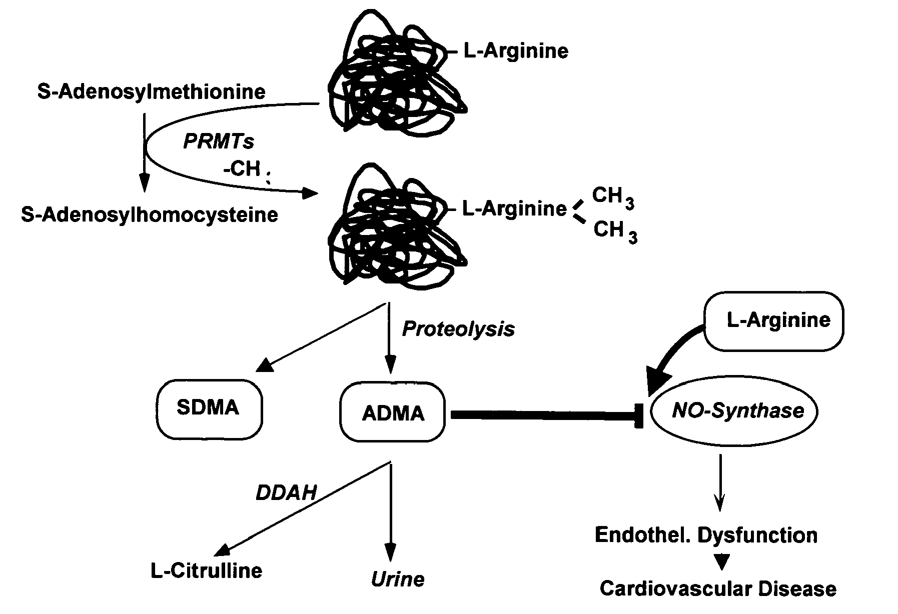

Imbalance of NO supply and requirement may be the initial step for pathological changes. Accumulating evidence links such imbalance of homeostasis to ADMA. Thus far, circulating ADMA was shown to be altered in patients suffering from cardiovascular and neurological disease as well as erectile dysfunction. Elevated plasma ADMA concentrations are found in various clinical settings ranging from renal failure to atherosclerosis, hypertension diabetes, preeclampsia, alzheimer's disease and even depression or schizophrenia.

Moreover, in patients with cardiovascular or renal disease elevated plasma ADMA concentrations independently predict progression of atherosclerosis and mortality. So far, causality in cardiovascular disease is considered likely but not definitely proven: Infusion of ADMA ameliorates endothelium-dependent vasodilation in humans.

The fact that life has evolved a highly specific enzymatic mechanism for ADMA degradation provides further evidence for a direct (patho-) physiological role of ADMA. It is therefore important to further examine the role of ADMA, which requires an analytical method capable of a high sample throughput.

In the state of the art, methods for determination of ADMA, SDMA and ARG have been described like spectrophotometry, capillary electrophoresis (CE) and assays based on ELISA, HPLC, MS and tandem-MS.

Current analytical methods for the measurement of ADMA focus on the use of HPLC-MS or HPLC-MS/MS. For example, Martens-Lobenhoffer et al. (Martens-Lobenhoffer J, Bode-Böger SM., J Chromatogr B Analyt Technol Biomed Life Sci., 2003 Dec. 251; 798(2), 231-39) used o-phthaldialdehyde (OPA) - 2-mercaptoethanol derivatives of ADMA, SDMA and ARG for enhancing the chromatographic separation of the analytes. This was crucial since the analytes undergo similar dissociations leading to a very similar tandem-MS spectra so that this more specific detection method could not be used. Instead, Martens-Lobenhofer et al. used MS in the full scan single mass spectrometry mode. Total analysis time was over 32 minutes, which is too long for high throughput or screening analysis. Another disadvantage of the method applied by Martens-Lobenhofer et al. is the poor stability of the OPA derivatives of the analytes, which therefore have to be analysed on-line.

Vishwanathan et al. (Vishwanathan K, Tacket RL, Stewart JT, Bartlett MG; J Chromatogr B Biomed Sci Appl., 2000 Oct. 1; 748(1), 157-66) omitted the step of derivatization and analysed the nonderivatized free amino acids. They separated the amino acids by means of HPLC using a silica column. Although they were able to use the tandem-MS, Vishwanathan et al., found that ADMA and SDMA show a very similar MS-MS spectra due to their very similar dissociation reactions. For example, the m/z of 158 used for the quantification for ADMA (203→158) was also observed for SMDA, ARG and MMA with relative intensities of 20%, 10% and 20%, respectively. Taking into account that these analytes are endogenous and that the concentration of SDMA is similar to that of ADMA and that of ARG up to ten times higher than that of ADMA, a severe disturbance of the quantification of ADMA at m/z 158 will occur, if the analytes are analysed simultaneously. Therefore, the time consuming chromatographic separation was essential for the method applied by Vishwanathan et al. A further disadvantage of this method are the difficulties in achieving the separation of the positional isomers ADMA and SDMA. The total analysis time is between 15 and 20 minutes, which is too long for fast routine analysis.

Huang et al. (Huang LF, Guo FQ, Liang YZ, Li BY, Cheng BM; Anal Bioanal Chem; 2004 Sep. 24; epub ahead of print) also omitted the step of derivatization to expedite the analytic process. They used LC-MS to analyse the analytes as free amino acids. Chromatographic separation was achieved using a RP18 column and again was crucial, since ADMA and SDMA showed a similar mass spectra. The total analysis time was longer than 7 minutes, which however is still not suitable for routine analysis. Moreover, in a plasma sample analysed by the Huang et al., a baseline separation could not be achieved as can be seen from fig. 3 of this paper. This results in an incorrect quantification. In addition, the applied isocratic separation will lead to accumulation of sample matrix on the column in case of high throughput analysis methods.

According to the state of the art, a simultaneous detection appears not to be possible and chromatographic separation seems to be crucial for unambiguously determining the concentration of these analytes. Although the step of derivatization is omitted, analysis time is still to long to carry out screening experiments. Summing up, in the state of the art no suitable high throughput analysis method is described. This limits the improvement of ADMA-research, and thus limits the medical and pharmaceutical progress.

It is object of the invention to overcome the limitations of the state of the art and to develop a methodology to detect ARG and its derivatives (i.e: MMA, ADMA and SDMA) with greater sensitivity, specificity and with higher throughput.

Before the present invention is further described, it is to be understood that this invention is not limited to particular embodiements described, as such may, of course, vary. It is also to be understood that the terminology used herein is for the purpose of describing particular embodiements only and is not intended to be limiting, since the scope of the present invention will be limited only by the appended claims.

The methods of the invention may be qualitative or quantitative. Thus, as used herein, the term "detection" refers to both qualitative and quantitative determinations, and therefore includes "measuring" and "determining a level of". A "sample" encompasses a variety of sample types obtained from an individual and can be used in a diagnostic or monitoring sense. The definition encompasses blood, blood-derived samples and other liquid samples of biological origin, tissue samples such as a biopsy specimen or tissue cultures or cells derived therefrom and the progeny thereof. That also includes samples that have been manipulated in any way after their procurement, such as by treatment with reagents, solubilization, or enrichment for certain components. It encompasses clinical samples and also cells in culture, cell supernatants, cell lysates, serum, plasma, cerebrospinal fluid, urine, saliva, biological fluidtissue samples, and dietary compounds and products.

Where a range of values is provided, it is understood that each intervening value is encompassed within the invention, likewise the upper and lower limits. Where the stated range includes one or both of the limits, ranges excluding either or both of those included limits are also included in the invention.

It must be noted that as used herein and in the appended claims, the singular forms "a", "and", and "the" include plural reference and vice versa unless the context clearly dictates otherwise.

The publications discussed herein are provided solely for their disclosure prior to the filing date of the present application. Nothing herein is to be construed as an admission that the present invention is not entitled to antedate such publication by virtue of prior invention.

The present invention provides methods of detecting one or more analytes of the group formed of arginine, its monomethylated derivatives, its dimethylated derivatives and its other derivatives in biological samples, particularly in samples that may contain ARG, ADMA, SDMA and MMA. The structures of ARG, ADMA, SDMA and MMA are shown below. 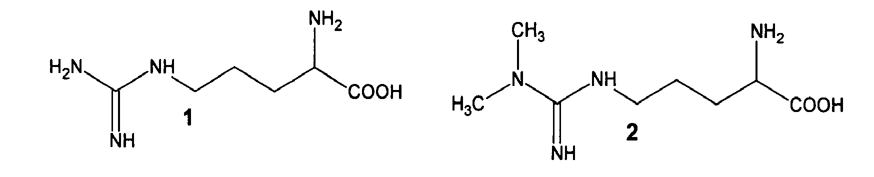 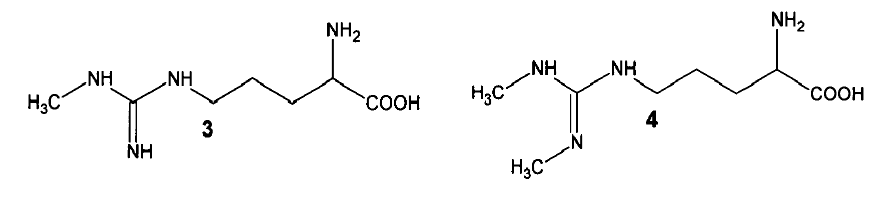 1: Arginine, 2: ADMA, 3: MMA, 4: SDMA

The methods generally involve derivatization and detection. The object of the present invention is achieved by the fact that the obtained derivatives of the analytes allow for simultaneous mass spectroscopical detection, preferably tandem mass spectroscopical detection. Simultaneous mass spectroscopic detection allows omitting or at least distinctively shortening the time consuming step of chromatographic separation prior to detection and thereby expediting the analytical process. As described above, the state of art obviously tried to shorten analysis time by omitting the derivatization step. In contrast to that, the present invention returns to this out-dated procedure and surprisingly achieves the objects by doing so.

As cited in the state of the art by Martens-Lobenhoffer et al. and Vishwanathan et al., ADMA and SDMA show a very similar fragmentation pattern in MS, especially in tandem-MS so that the use of this highly specific and sensitive method for simultaneous detection of the two analytes was found not to be suitable and chromatographic separation of the analytes became crucial and had to be applied prior to detection. The present invention achieves the objects of the invention by derivatizing the analytes in a way that the derivatized analytes exhibit a different fragmentation pattern with unambiguous and characteristic mass signals that are not disturbed by the signals originating from the other analytes. Thus, there is no longer a need for a thorough chromatographic separation, the derivatized analytes may be distinguishably detected simultaneously instead. Thereby, MS and all combinations of MS, for example tandem-MS, ion trap-MS, MS-TOFLMS or further MSⁿ-techniques are applicable and within the scope of this invention.

It is especially useful, if each mother ion of the derivatized analytes generated within the analytical process is fragmented at least a second time with the resulting spectra differing from the spectra of the other mother ions. In this way, a highly specific analysis is possible since the daughter ions of one mother ion are different from the daughter ions of another mother ion. Therefore, detection uses the typical fragmentation pattern of each mother ion or if necessary of each daughter ion. This qualifies the method according to the invention to be used in combination with any MS, for example tandem-MS, ion trap-MS, MS-TOFLMS or further MSⁿ-techniques.

Although one may wish to apply chromatographic separation in some cases to exclude the biological matrix from entering the detector, it is not indispensably necessary. In the embodiements of the invention, where a step of separation is provided, it is preferably used for separating the derivatized analytes from the biological matrix, preferably by using HPLC or CE and not the analytes themselves. A distinct baseline separation of the derivatized analytes is not essential, so that even in these embodiements, the analytical process proceeds faster than disclosed in the state of the art.

The derivatization according to the invention comprises the derivatization of the carboxylic acid function of the analytes, preferably by acid esterification or by amidation. For the esterification, any suitable alcohol may be used, especially linear or branched alcyl alcohols, substituted or not, olefinic, aromatic or organic alcohols or phenols. For amidation, all suitable reagents known to the one skilled in the art may be used. Preferably, suitable nitrogen compound may be used, preferably NR₃ with R being hydrogen or any hydrocarbon, whereas each R may differ from the other. Especially the use of diethylamine turned out to be very useful. Derivatization at this function of the analytes surprisingly results in distinguishable fragmentation pattern of the analytes. Advantageously, derivatizing this function is easy to handle and results in stable derivatives. This is an important difference to the state of the art derivatization with the well established OPA at the amino-function of the amino acid. Any other derivatization that will lead to derivatives having different fragmentation patterns of mother or daughter ions are within the scope of this invention.

In an embodiement of the invention it is provided that the derivatization is conducted in any automated fashion known to the person skilled in the art. Although this will result in additional costs for providing the analytical apparatus, a further expedition of the total analytical process can advantageously be achieved by this feature.

Depending upon the nature of the samples, in some embodiements of the invention, a sample preparation is provided, preferably consisting of a protein precipitation, particularly a solvent precipitation which is easy to handle and not disturbing the MS, especially tandem-MS, detection. Preferably, this optional step is automated. Automation can be achieved by using any method known to the person skilled in the art.

Advantageously, the methods disclosed in the present invention may make use of a wide spectrum of ionisation techniques for the step of detection. These techniques comprise ESI, APCI or other appropriate techniques known to the one skilled in the art. Thus, the disclosed method is readily available for many laboratories or investigators due to the fact that many laboratories already posess such ionisation techniques as ESI.

For reason of quality management and control, a calibration is provided making use of an internal standard and/or labelled standards. The latter are preferably stable isotopes, for instance ²H, ¹³C, ¹⁵N, ¹⁷O, ¹⁸O, or any other stable isotope known to the person skilled in the art. Advantageously, the method applies internal standards for quantification and uses them to calculate the ratio of any analyte to any other analyte, preferably the ADMA over SDMA ratio, the L-arginine over ADMA ratio, and reciprocal ratios. Therefore, the analytes themselves in a deuterated form are used since a chromatographic separation is not necessary. By doing so, calibration process is simplified and easy to handle.

### Example 1

Samples of human plasma, urine and cell supernatants were analysed quantitatively. 50 µL sample were diluted with L-[²H₇]-arginine and [²H₆]-ADMA to concentrations of 50 and 2 µmol/L, respectively, followed by subsequent protein precipitation with 100 µL of acetone. The supernatant was dried and taken up into 100 µL of a 1 M hydrochloric acid solution in butanol. Derivatization was conducted for 17 min at 65°C. After evaporation samples were reconstituted in 1 mL of water.

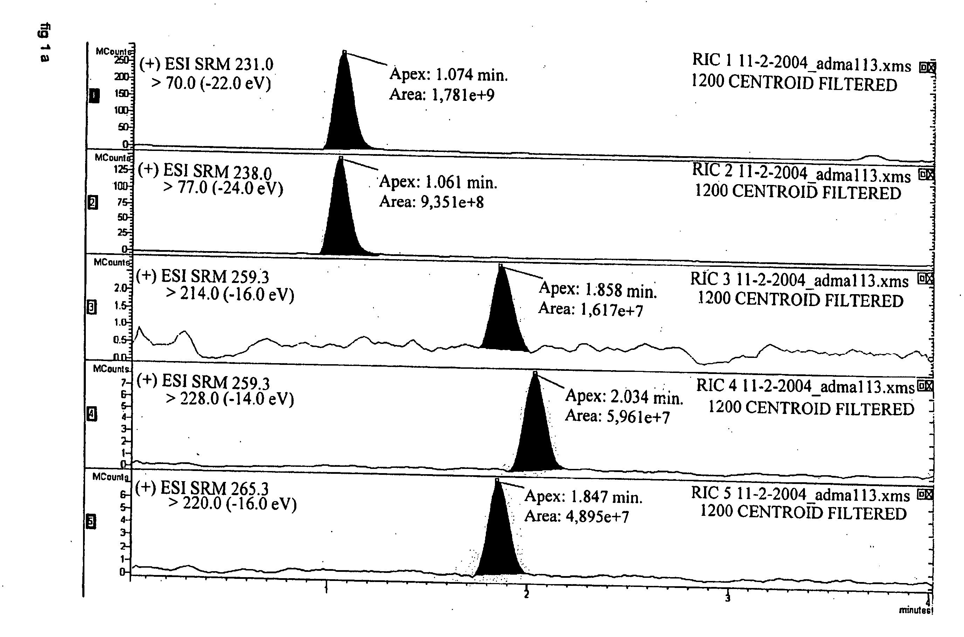

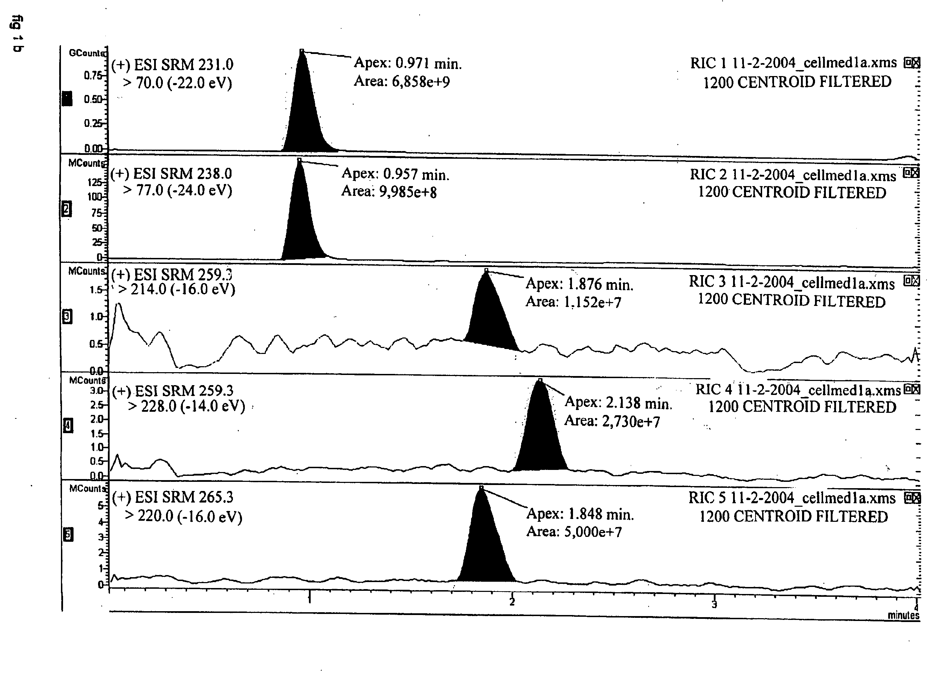

Chromatographic separation was conducted on an analytical column [50 X 2.0 mm (i.d.)] packed with Polaris C₁₈-Ether 3 µ. The column was kept at 25 °C. The mobile phase consisted of methanol (0.1 % formic acid, phase A) and water (0.1 % formic acid, phase B). A gradient was pumped at a flow rate of 0.4 mL/min starting with 2 % of A for 0.5 min followed by a linear increase to 50 % of A for 1.5 min and subsequent equilibration at 2 % of A for 2 min. Retention times can be taken from fig. 1. The analytes eluted all within a period of approximately 1 minute beginning from t= 0.8 min so that the whole chromatographic separation process needs not more than about 2 minutes. Shortening of the retention time is easily achievable, resulting in a very fast analytical process.

LC-tandem MS analyses were performed on a Varian 1200L Triple Quadrupole MS. Main gas, drying gas (380 °C), and nebulizing gas (N₂) were pumped at 80, 24, and 60 psi, respectively. For ionisation in the ESI+ ionisation mode needle and shield voltage were set at 5800 and 400 Volts, respectively. Argon was used for MS/MS fragmentation at a collision cell pressure of 1.5 mTorr. The following transitions were analysed: mass-to-charge ratio (m/z) 231 → m/z 70 (collision energy (CE) -22 Volts) for L-arginine; m/z 238 → m/z 77 (CE -24 Volts) for L-[²H₇]-arginine (98 atom% ²H isotopic purity); m/z 259 → m/z 214 (CE -16 Volts) for ADMA; m/z 259→ m/z 228 (CE -14 Volts) for SDMA; and m/z 265→ m/z 220 (CE -16 Volts) for [²H₆]-ADMA (98 atom% ²H isotopic purity). All compounds were analysed as their butyl ester derivatives. A brief scheme of the possible fragmentation of ARG, ADMA and SDMA is given below:

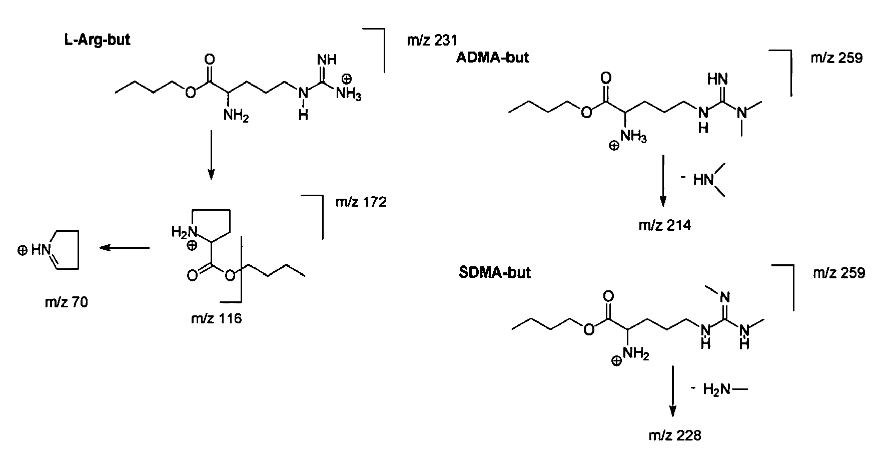

Without wishing to be bound by theory, it is believed that the hindrance of the formation of an intermediate intramolecular complex by the nature of derivatization is crucial for the invention.

Fig. 1a-b gives examples of a LC-MS/MS chromatogram of a human plasma sample and a LC-MS/MS chromatogram of a cell culture supernatant sample according to example 1. From top to bottom of each diagram, the analytes arginine, [²H₇]-arginine as internal standard, ADMA, SDMA and [²H₆]-ADMA as internal standard are shown. It can clearly be seen that methylated arginines elute at the same time and therefore have to be and are analyzed simultaneously.

For reasons of calibration, solutions of synthetic L-arginine and ADMA were used in the range 0-100 µmol/L for L-arginine and 0-4 µmol/L for ADMA. L-[²H₇]-arginine and [²H₆]-ADMA were added as internal standards at concentrations of 50 and 2 µmol/L, respectively. The linear regression equations for peak area ratio (y) and ratio of injected calibrators (x) were: y = 0.93 x + 0.018 (*r*² = 0.999) for L-arginine and y = 0.93 x + 0.017 (*r*² = 0.999) for ADMA. Peak area ratios obtained were corrected for the different response factors for unlabeled and labeled compounds obtained.

A validation of the method for the quantitative determination of L-arginine and ADMA in human and mouse plasma as well as cell culture supernatants was conducted by adding L-arginine and ADMA in duplicate to samples. L-arginine was added at 0, 12.5, 25, 50, and 100 µmol/L. ADMA was added at 0, 0.5, 1, 2, and 4 µmol/L. Linear regression analysis between measured (*y*) and added (*x*) L-arginine concentration yielded a slope of 1.09 and y-axis intercept of 26.2 µmol/L with *r*² = 0.996 for human plasma. Similar equations were obtained for mouse plasma and cell culture supernatants (human coronary artery endothelial cells). Linear regression analysis between measured (*y*) and added (x) ADMA concentration yielded a slope of 1.03 and y-axis intercept of 0.39 µmol/L with *r*² = 0.999 for human plasma. Similar equations were obtained for mouse plasma and cell culture supernatants.

The limit of detection of the method for ADMA was tested. It could be shown, that signal-to-noise ratio observed for 0.05 pg/µL ADMA was 16:1 whereas 0.01 pg/µL ADMA as the lowest applied concentration was not detectable.

### Example 2

The same methodology is applied as outlined for example 1 including the following modifications: Samples of human plasma, urine and cell supernatants were analysed quantitatively. 50 µL sample were diluted with L-[²H₇]-arginine and [²H₆]-ADMA to concentrations of 50 and 2 µmol/L, respectively, followed by subsequent protein precipitation with 100 µL of acetone. The supernatant was dried and taken up into 100 µL of a 2M hydrochloric acid solution in methanol. Derivatization was conducted for 60 min at 80°C. After evaporation samples were reconstituted in 1 mL of water. LC-tandem MS analyses were performed on a Varian 1200L Triple Quadrupole MS. Main gas, drying gas (300 °C), and nebulizing gas (N₂) were pumped at 80, 24, and 60 psi, respectively. For ionisation in the ESI+ ionisation mode needle and shield voltage were set at 5000 and 400 Volts, respectively. Argon was used for MS/MS fragmentation at a collision cell pressure of 1.5 mTorr. The following transitions were analysed: mass-to-charge ratio (m/z) 189 → m/z 70 (collision energy (CE) -22 Volts) for L-arginine; m/z 196 → m/z 77 (CE -24 Volts) for L-[²H₇]-arginine; m/z 217 → m/z 172 (CE -16 Volts) for ADMA; m/z 217→ m/z 186 (CE -14 Volts) for

SDMA; and m/z 223 → m/z 178 (CE -16 Volts) for [²H₆]-ADMA. All compounds were analysed as their methyl ester derivatives.

### Example 3

The same methodology is applied as outlined for example 1 including the following modifications: Samples of human plasma, urine and cell supernatants were analysed quantitatively. 50 µL sample were diluted with L-[²H₇]-arginine and [²H₆]-ADMA to concentrations of 50 and 2 µmol/L, respectively, followed by subsequent protein precipitation with 100 µL of acetone. The supernatant was dried and taken up into 100 µL of a 2M hydrochloric acid solution in ethanol. Derivatization was conducted for 30 min at 65°C. After evaporation samples were reconstituted in 1 mL of water. LC-tandem MS analyses were performed on a Varian 1200L Triple Quadrupole MS. Main gas, drying gas (300 °C), and nebulizing gas (N₂) were pumped at 80, 24, and 60 psi, respectively. For ionisation in the ESI+ ionisation mode needle and shield voltage were set at 5000 and 400 Volts, respectively. Argon was used for MS/MS fragmentation at a collision cell pressure of 1.5 mTorr. The following transitions were analysed: mass-to-charge ratio (m/z) 203 → m/z 70 (collision energy (CE) -22 Volts) for L-arginine; m/z 210 → m/z 77 (CE -24 Volts) for L-[²H₇]-arginine; m/z 231 → m/z 186 (CE -16 Volts) for ADMA; m/z 231 → m/z 200 (CE -14 Volts) for

SDMA; and m/z 237 → m/z 192 (CE -16 Volts) for [²H₆]-ADMA. All compounds were analysed as their ethyl ester derivatives.

### Example 4

The same methodology is applied as outlined for example 1 including the following modifications: Samples of human plasma, urine and cell supernatants were analysed quantitatively. 50 µL sample were diluted with L-[²H₇]-arginine and [²H₆]-ADMA to concentrations of 50 and 2 µmol/L, respectively, followed by subsequent protein precipitation with 100 µL of acetone. The supernatant was dried and taken up into 100 µL of a 2M hydrochloric acid solution in methanol. Derivatization was conducted for 60 min at 80°C. After evaporation samples were reconstituted in 100 µL diethylamine (free base). Reaction was performed for 60 min at 65°C. After evaporation samples were reconstituted in 1 mL of water. LC-tandem MS analyses were performed on a Varian 1200L Triple Quadrupole MS. Main gas, drying gas (300 °C), and nebulizing gas (N₂) were pumped at 80, 24, and 60 psi, respectively. For ionisation in the ESI+ ionisation mode needle and shield voltage were set at 5000 and 400 Volts, respectively. Argon was used for MS/MS fragmentation at a collision cell pressure of 1.5 mTorr. The following transitions were analysed: mass-to-charge ratio (m/z) 241 → m/z 70 (collision energy (CE) -22 Volts) for L-arginine; m/z 248 → m/z 77 (CE -24 Volts) for L-[²H₇]-arginine; m/z 269 → m/z 224 (CE -16 Volts) for ADMA; m/z 269 → m/z 238 (CE -14 Volts) for SDMA; and m/z 275 → m/z 230 (CE -16 Volts) for [²H₆]-ADMA.

## Claims

1. Method for detecting one or more analytes of the group formed by arginine, its monomethylated derivative monomethylarginine (MMA), its dimethylated derivatives asymmetric dimethylarginine (ADMA) and symmetric dimethylarginine (SDMA) in biological samples comprising the steps of derivatization and detection of analytes, being **characterized in that**
- the analytes are derivatized at their carboxylic acid function, preferably by acid esterification, particularly by means of methanol, ethanol and/or butanol, or by amidation, particularly by means of diethylamine;
- the obtained derivatives of analytes are detected simultaneously by mass spectroscopical detection, preferably tandem mass spectroscopical detection.

2. Method according to claim 1, being **characterized in that** the derivatization is conducted in any automated fashion.

3. Method according to any preceding claim, **being characterized in that** the mass spectrometric detection is achieved by single quadrupole, triple quadrupole, ion trap, ion cyclotron resonance, time-of-flight MS, or any other suitable MS system or combination of the aforementioned MS systems.

4. Method according to any preceeding, being **characterized in that** a step of separation is provided, which preferably separates the derivatized analytes from a biological matrix, preferably by using HPLC or capillary electrophoresis.

5. Method according to any preceding claim, being **characterized in that** a step of sample preparation is provided, preferably consisting of a protein precipitation, particularly a solvent precipitation, which preferably is conducted automated.

6. Method according to any preceeding claim, being **characterized in that** the detection comprises an ionisation technique of the group formed by ESI, APCI, MALDI.

7. Method according to any preceding claim, **being characterized in that** a calibration is provided making use of an internal standard and/or labelled standard and that it is used to calculate the ratio of any analyte to any other analyte, preferably the ADMA over SDMA ratio, the L-arginine over ADMA ratio, and reciprocal ratios.

## Patentansprüche

1. Verfahren zum Nachweisen von einem oder mehreren Analyten der aus Arginin, seinem monomethylierten Derivat Monomethylarginin (MMA) und seinen dimethylierten Derivaten asymmetrisches Dimethylarginin (ADMA) und symmetrisches Dimethylarginin (SDMA) gebildeten Gruppe in biologischen Proben, umfassend die Schritte des Derivatisierens und des Nachweisens von Analyten, **dadurch gekennzeichnet, dass**
- die Analyten an ihrer Carbonsäurefunktion derivatisiert werden, vorzugsweise durch Veresterung der Säure, insbesondere mithilfe von Methanol, Ethanol und/oder Butanol, oder durch Amidierung, insbesondere mithilfe von Diethylamin;
- die erhaltenen Derivate von Analyten gleichzeitig durch massenspektrometrischen Nachweis, vorzugsweise durch tandem-massenspektrometrischen Nachweis, nachgewiesen werden.

2. Verfahren nach Anspruch 1, **dadurch gekennzeichnet, dass** das Derivatisieren auf eine beliebige, automatisierte Weise durchgeführt wird.

3. Verfahren nach einem der vorangegangenen Ansprüche, **dadurch gekennzeichnet, dass** das massenspektrometrische Nachweisen durch Einfach-Quadrupol-, Dreifach-Quadrupol-, lonenfallen-, lonenzyklotronresonanz-, Flugzeit-MS oder ein beliebiges anderes, geeignetes MS-System oder durch eine Kombination aus den genannten MS-Systemen erzielt wird.

4. Verfahren nach einem der vorangegangenen Ansprüche, **dadurch gekennzeichnet, dass** ein Schritt des Trennens bereitgestellt ist, der vorzugsweise die derivatisierten Analyten von einer biologischen Matrix trennt, vorzugsweise durch die Verwendung von HPLC oder Kapillarelektrophorese.

5. Verfahren nach einem der vorangegangenen Ansprüche, **dadurch gekennzeichnet, dass** ein Schritt des Probenvorbereitens bereitgestellt ist, der vorzugsweise aus einer Proteinfällung, insbesondere einer Lösungsmittelfällung, besteht, die vorzugsweise automatisiert durchgeführt wird.

6. Verfahren nach einem der vorangegangenen Ansprüche, **dadurch gekennzeichnet, dass** das Nachweisen ein lonisierungsverfahren der aus ESI, APCI und MALDI gebildeten Gruppe umfasst.

7. Verfahren nach einem der vorangegangenen Ansprüche, **dadurch gekennzeichnet, dass** eine Kalibrierung bereitgestellt ist, die eine interne Standardsubstanz und/oder markierte Standardsubstanz benutzt, und dass es dazu verwendet wird, das Verhältnis eines Analyten zu einem anderen Analyten zu berechen, vorzugsweise das Verhältnis von ADMA zu SDMA, das Verhältnis von L-Arginin zu ADMA und umgekehrte Verhältnisse.

## Revendications

1. Procédé pour détecter un ou plusieurs analytes parmi le groupe formé par arginine, son dérivé monométhylé monométhylarginine (MMA), ses dérivés diméthylés diméthylarginine asymétrique (ADMA) et diméthylarginine symétrique (SDMA) dans des échantillons biologiques, comprenant les étapes de dérivatisation et de détection d'analytes, **caractérisé par le fait que**
- les analytes sont dérivatisés à leur fonction d'acide carboxylique, de préférence par estérification, en particulier au moyen de méthanol, d'éthanol et/ou de butanol, ou par amidation, en particulier au moyen de diéthylamíne;
- les dérivés d'analytes obtenus sont détectés simultanément par détection spectroscopique de la masse.

2. Procédé selon la revendication 1, **caractérisé par le fait que** la dérivatisation est réalisée de toute manière automatisée.

3. Procédé selon l'une ou l'autre revendication précédente, **caractérisé par le fait que** la détection spectroscopique de la masse est réalisée par un système SM quadripôle simple, quadripôle triple, à piège ionique, à résonance cyclotronique ionique, à temps de vol ou tout autre système SM approprié ou combinaison des systèmes SM précités.

4. Procédé selon l'une ou l'autre revendication précédente, **caractérisé par le fait qu'**il est prévu une étape de séparation qui sépare de préférence les analytes dérivatisés d'une matrice biologique, de préférence à l'aide de HPLC ou d'électrophorèse capillaire.

5. Procédé selon l'une ou l'autre revendication précédente, **caractérisé par le fait qu'**il est prévu une étape de préparation d'échantillon consistant, de préférence, en une précipitation de protéine, en particulier une précipitation de solvant, qui est, de préférence, réalisée de manière automatisée.

6. Procédé selon l'une ou l'autre revendication précédente, **caractérisé par le fait que** la détection comprend une technique d'ionisation du groupe formé par ESI, APCI, MALDI.

7. Procédé selon l'une ou l'autre revendication précédente, **caractérisé par le fait qu'**il est prévu un étalonnage utilisant un standard interne et/ou un standard étiqueté et qui est utilisé pour calculer le rapport entre tout analyte et tout autre analyte, de préférence le rapport entre ADMA et SDMA, le rapport entre L-arginine et ADMA, et des rapports réciproques.
